# DEMANDE DE BREVET EUROPEEN

(11) **EP 1 095 666 A1**
(43) Date de publication de la demande: **02.05.2001**
(21) Numéro de dépôt: 99810984.7
(22) Date de dépôt: 29.10.1999
(51) Int. Cl.: A61M 1/34

(54) **Dispositif d'épuration extracorporelle du sang**

(71) Demandeur: Infomed S.A., 1227 Genève (CH)
(72) Inventeur: Favre, Olivier, 1208 Geneve (CH); Di Lella, Francesco, 1205 Geneve (CH)
(74) Mandataire: Savoye, Jean-Paul

(57) **Abrégé**

Ce dispositif d'épuration extracorporelle du sang comprend un conduit d'extraction du sang (1), un conduit de retour du sang (3), des moyens de filtration du sang (4), un conduit d'évacuation de l'ultrafiltrat (5) issu desdits moyens de filtration (4), une source de solution de substitution (S), deux conduits de liaison (7', 7") entre cette source (S) et d'une part, ledit conduit d'extraction du sang (1), d'autre part, ledit conduit de retour du sang (3). Il comporte en outre des moyens de mesure (10-13) d'au moins un des paramètres influencés par la résistance des moyens de filtration (4) à l'écoulement du liquide, des moyens pour déterminer au moins une valeur de seuil dudit paramètre, des moyens pour comparer ledit paramètre avec ladite valeur de seuil, des moyens de commande d'échange pour calculer la répartition des débits respectifs à travers lesdits conduits de liaison (7', 7"), apte à réduire l'écart entre la valeur dudit paramètre mesuré et ladite valeur de seuil.

## Description

La présente invention se rapporte à un dispositif d'épuration du sang comprenant un conduit d'extraction du sang, un conduit de retour du sang, des moyens de filtration du sang situés entre le conduit d'extraction et le conduit de retour, des moyens pour faire circuler le sang, un conduit d'évacuation du liquide issu desdits moyens de filtration appelé ultrafiltrat, des moyens pour faire circuler l'ultrafiltrat dans ledit conduit d'évacuation, une source de solution de substitution, deux conduits de liaison entre cette source et d'une part, ledit conduit d'extraction du sang, d'autre part, ledit conduit de retour du sang et des moyens pour faire circuler la solution de substitution dans chacun desdits conduits de liaison.

L'épuration extracorporelle a pour but d'une part de nettoyer le sang des patients en retirant des éléments indésirables et d'autre part de contrôler le poids des patients. L'invention s'applique plus particulièrement à l'hémofiltration qui se distingue de la dialyse par le fait que l'épuration s'opère par convection plutôt que par diffusion à travers une membrane semi-perméable. Dans les deux cas l'opérateur est appelé à intervenir durant les séances, en particulier pour éviter la coagulation dans le circuit de circulation extracorporelle, qui se produit notamment au niveau du filtre.

La coagulation de la circulation extracorporelle en hémofiltration est classiquement réduite en employant des anti-coagulants (héparine, liquémine) et en procédant à intervalles réguliers à un rinçage du circuit et au changement des filtres.

Souvent bien tolérées par les patients dans les doses habituelles, l'utilisation d'anti-coagulants est contre-indiquée dans certains cas, par exemple pour les patients comportant des lésions importantes.

Quant au rinçage, c'est une opération qui consiste à faire momentanément circuler du liquide physiologique dans le filtre à la place du sang. Cela s'effectue en obstruant le conduit d'extraction du sang avec un clamp, en reliant une poche de liquide physiologique au conduit d'extraction du sang, en attendant qu'une quantité suffisante de liquide physiologique se soit écoulée, habituellement de 100 à 300 ml. Cette quantité est considérée comme apte à nettoyer le filtre. On rétablit ensuite les conditions d'écoulement antérieures pour poursuivre le traitement. Cette suite d'opérations prend un temps important et peut mener à des erreurs de manipulation. De plus, elle interrompt le traitement et l'opérateur doit tenir compte du surplus de liquide injecté au patient dans le calcul du bilan hydrique de ce dernier.

Au cas où un changement de filtre est nécessaire, il faut retourner son sang au patient, rincer le nouveau filtre puis poursuivre le traitement. Cette opération est longue et coûteuse, il faut donc l'éviter autant que possible.

Notons encore qu'au phénomène de coagulation s'ajoute souvent celui d'encrassement qui est un dépôt de molécules sur les surfaces de la circulation extracorporelle et notamment les parois du filtre. Cet encrassement réduit également les capacités d'épuration du sang et est éliminé de la même façon que les dépôts coagulés. Dans la suite du texte on emploie indifféremment l'un des termes coagulation ou encrassement pour désigner les deux phénomènes.

On a proposé dans le WO 83/04373 un appareil pour réaliser automatiquement des rinçages en actionnant des clamps électromécaniques à des intervalles de temps prescrits par l'opérateur. Le brevet EP 0 235 591 propose d'ajouter un clamp qui permet de faire varier la pression dans le filtre afin d'augmenter l'effet du rinçage. L'inconvénient de ces deux solutions reside dans le fait que ces rinçages sont décidés par l'utilisateur qui n'a qu'une idée approximative de la vitesse de coagulation du système. Par conséquent, soit le rinçage est trop fréquent, ce qui diminue l'efficacité du traitement par des interruptions répétées de ce dernier, soit il n'est pas assez fréquent, conduisant à une coagulation par insuffisance de rinçage.

Dans d'autres appareils connus, la totalité du liquide de remplacement est apportée en prédilution, augmentant ainsi le débit et la dilution du sang dans le filtre. Ceci a pour effet de réduire la coagulation du système, mais d'augmenter considérablement la quantité de liquide de substitution consommé et le temps de traitement. On sait en effet, qu'à des débits élevés, au moins 30% du volume injecté en prédilution traverse le filtre sans participer à l'épuration du sang. Ainsi, avec un traitement nécessitant par exemple un volume d'échange de 45 litres, la substitution étant entièrement en prédilution, 15 litres d'une solution physiologique coûteuse passent inutilement à travers le filtre.

Notons que le volume d'échange, auquel correspond un débit d'échange, est défini comme étant le volume de liquide participant à l'épuration du sang retiré de ce dernier au cours de la séance. Si l'on n'a pas de pertes, notamment par prédilution, ce volume correspond à celui d'ultrafiltrat. C'est la donnée qui, en hémofiltration, détermine le degré d'épuration du sang au cours de la séance, lequel pourrait aussi être défini comme étant le quantité d'impuretés retirées du sang.

Aucun des systèmes cités précédemment n'est réellement satisfaisant puisqu'ils ne s'adaptent pas aux variations des paramètres de fonctionnement qui peuvent apparaître durant le traitement. De plus, les patients sont régulièrement épurés de façon insuffisante du fait que les volumes prescrits sont calculés en admettant que la totalité du liquide de substitution est injectée en postdilution, les valeurs n'étant pas corrigées pour tenir compte de la proportion de ce liquide introduit en prédilution.

Les paramètres physiques qui peuvent avoir une influence sur la coagulation du système sont nombreux. On peut notamment citer la matière et la surface du filtre, la composition et le débit sanguin, la dose d'anticoagulant prescrite et le débit d'échange souhaité. Ces paramètres peuvent varier au cours du traitement dans des proportions importantes et des délais courts. Il est donc impossible pour l'utilisateur, de contrôler l'état du système de manière à éviter systématiquement sa coagulation. Ceci est d'autant plus vrai que la durée des traitements peut être de plusieurs jours et que les débits d'échange sont importants, par exemple de douze litres par heure.

Le but de la présente invention est de remédier, au moins en partie, aux inconvénients susmentionnés.

A cet effet, cette invention a pour objet un dispositif d'épuration extracorporelle du sang du type susmentionné, selon la revendication 1.

Un avantage essentiel de cette invention réside dans le fait que ce dispositif ne nécessite pas d'intervention de l'utilisateur pendant le traitement.

Le dispositif selon l'invention permet de minimiser la solution de substitution consommée et d'adapter le volume d'échange de façon à respecter, dans la mesure du possible, toutes les consignes données par l'utilisateur.

L'invention sera mieux comprise à l'aide de la description qui suit et des dessins annexés, qui illustrent schématiquement et à titre d'exemple une forme d'exécution du dispositif objet de cette invention.

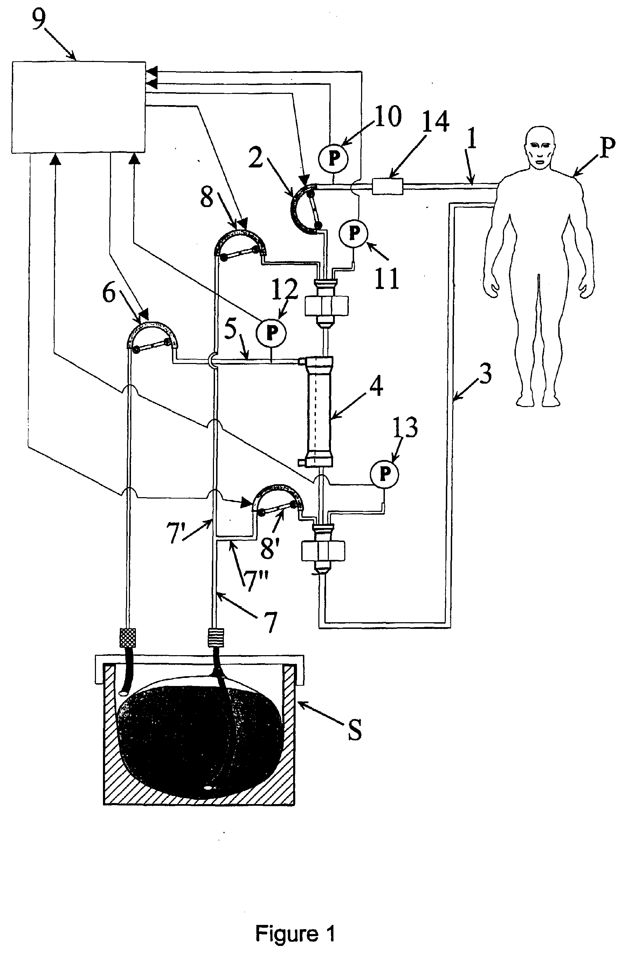

La figure 1 représente un schéma de cette forme d'exécution;
la figure 2 est un schéma bloc des moyens de calcul 9;
la figure 3 illustre la suite d'opérations pour la mise en marche du dispositif;
la figure 4 illustre la suite d'opérations pour le réglage des débits d'ultrafiltrat et de liquide de substitution, intégrant le calcul du nouveau volume d'échange rendu nécessaire par l'utilisation de la prédilution;

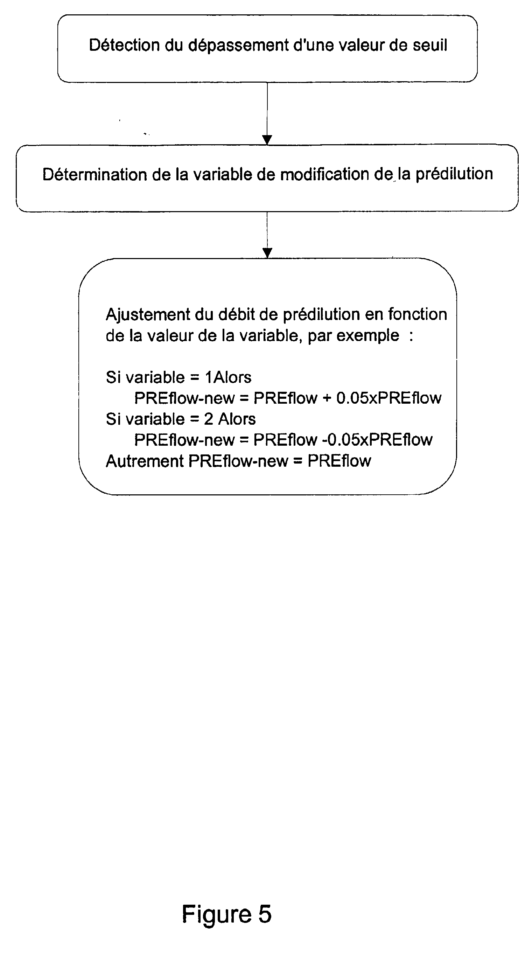

la figure 5 illustre la suite d'opérations pour le réglage du taux de prédilution.

L'invention possède des moyens de circulation extracorporelle du sang composés d'un conduit d'extraction 1, d'une pompe 2 pour extraire le sang du corps du patient P et d'un conduit de retour 3 pour ramener le sang épuré dans le corps du patient P. Un filtre 4 permet de réaliser l'épuration du sang, grâce à un conduit d'évacuation de la solution polluée 5, appelée ultrafiltrat, possédant un moyen de commande de débit 6.

Un conduit de solution de substitution 7 relie un réservoir de solution de substitution S au circuit de circulation extracorporelle 1, 3. Ce conduit se divise en deux conduits 7' et 7", le premier 7' reliant la source de liquide de substitution S au conduit d'extraction du sang 1 en amont du filtre 4, le second 7" reliant cette source S au conduit de retour 3, en aval du filtre 4. Le débit de liquide de prédilution à travers le conduit 7' est contrôlé par une pompe péristaltique 8. Le débit de liquide de postdilution à travers le conduit 7" est contrôlé par une pompe péristaltique 8'. Le liquide de prédilution peut indifféremment être injecté en amont ou en aval de la pompe 2 d'extraction du sang.

Des moyens de calcul 9 servent à déterminer la proportion du débit de liquide de prédilution et celle de liquide de postdilution. Des capteurs 10, 11, 12, et 13, de pression ou de débit sont disposés en différents endroits du circuit de circulation du sang 1, 3 et du conduit d'évacuation 5 de l'ultrafiltrat. Ces capteurs 10-13 sont reliés à l'unité de calcul 9 pour lui fournir les paramètres nécessaires à la détermination des quantités respectives des débits de liquide de substitution à envoyer dans les conduits 7' et 7" et correspondant aux valeurs de prédilution et de postdilution calculées.

Les composants habituels des dispositifs d'épuration extracorporelle qui n'interviennent pas directement dans le champ de la présente invention ne sont pas représentés. Ce sont notamment les détecteurs de bulles d'air, le clamp de fermeture de la ligne de retour 3 du sang, le détecteur de fuite de sang, le réchauffeur de sang ou de liquide de substitution et les moyens de mesure des masses écoulées.

Les moyens de commande des débits sont typiquement des pompes péristaltiques ou des clamps commandés par l'unité de calcul 9.

L'invention s'applique également aux systèmes de circulation extracorporelle à aiguille unique et aux appareils de dialyse intégrant ou non la fabrication des solutions, ainsi qu'aux méthodes combinées comme l'hémodiafiltration.

L'évolution de la coagulation du filtre peut être suivie par mesure de la différence de pression du sang entre l'entrée et la sortie du filtre 4, la pression transfiltre, calculée en effectuant la différence des valeurs fournies par les capteurs 11 et 13, et/ou par la pression transmembranaire Pₜₘ usuellement définie comme étant la différence de pression entre la pression P₁₂ du conduit d'ultrafiltrat mesurée par le capteur 12 et la pression moyenne P_{moy} calculée à partir des valeurs des capteurs 11 et 13, soit Pₜₘ = ( (P₁₁ + P₁₃) / 2) - P₁₂.

Pour atteindre les buts de traitement en évitant la coagulation du filtre 4, un système selon l'invention adapte les débits de liquide de substitution dans les conduits 7', 7" de manière à maintenir la valeur des paramètres influencés par ce phénomène dans leur valeurs normales de fonctionnement et adapte le volume d'échange, et par conséquence le débit d'ultrafiltrat, en tenant compte du débit de liquide de substitution qui s'est effectivement écoulé dans le conduit de prédilution 7', de manière à corriger sa valeur en déduisant la part de liquide de substitution n'ayant pas participé à l'épuration. De plus, le système minimise le volume de liquide de substitution qui s'écoule dans le conduit de prédilution 7', afin d'éviter le gaspillage de ce liquide de substitution et d'atteindre un meilleur degré d'épuration dans le temps donné.

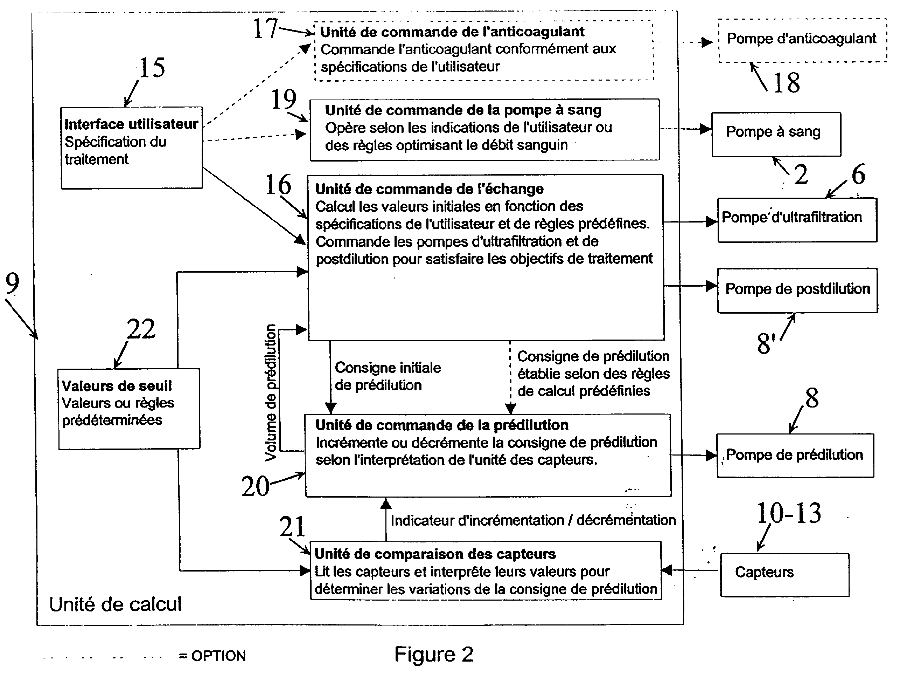

Le schéma-bloc de l'unité de calcul 9 illustrée par la figure 2 comporte une interface 15 à l'aide de laquelle l'opérateur peut introduire les spécifications relatives au traitement. Cette interface 15 est connectée à une unité de commande de l'échange 16 qui comporte un programme de calcul pour commander la pompe d'ultrafiltrat 6 et la pompe de postdilution 13 selon les indications provenant entre autres de l'interface 15, ainsi que de données et/ou de règles de calcul qui peuvent être contenues dans une mémoire (non représentée).

L'interface 15 peut de plus être reliée à une unité de commande 17 d'un poste de distribution d'anticoagulant 18, ainsi qu'à une unité de commande 19 de la pompe d'extraction du sang 2 qui agit en fonction des indications de l'opérateur ou de règles d'optimisation d'écoulement prédéfinies.

Le calculateur 9 comporte encore une unité de commande de la prédilution 20 reliée d'une part à l'unité de commande de la valeur d'échange 16 et à une unité d'interprétation 21 des mesures effectuées par les capteurs 10-13 et d'autre part à la pompe de prédilution 8. L'unité de commande de la prédilution 20 établit la valeur initiale du taux de prédilution en fonction des instructions reçues de l'unité de commande de la valeur d'échange 16 ou de règles ou valeurs préalablement enregistrées dans l'unité 20. Cette dernière augmente ou diminue le débit de la pompe de prédilution 8 en fonction de l'interprétation des valeurs mesurées par les détecteurs 10-13, que lui fournit l'unité d'interprétation 21. En variante, l'unité de commande de la valeur d'échange 16 fournit directement à l'unité de commande de la prédilution une valeur de consigne à appliquer à la pompe de prédilution 8, ladite valeur étant établie sur la base de règles de calcul enregistrées dans les moyens de calcul 9. Un exemple d'une telle règle consiste à maintenir la consigne de prédilution à zéro pour un débit d'ultrafiltrat inférieur à une valeur donnée, puis à augmenter ledit débit de prédilution selon une courbe proportionnelle à l'augmentation de l'ultrafiltrat au-dessus de ladite valeur donnée.

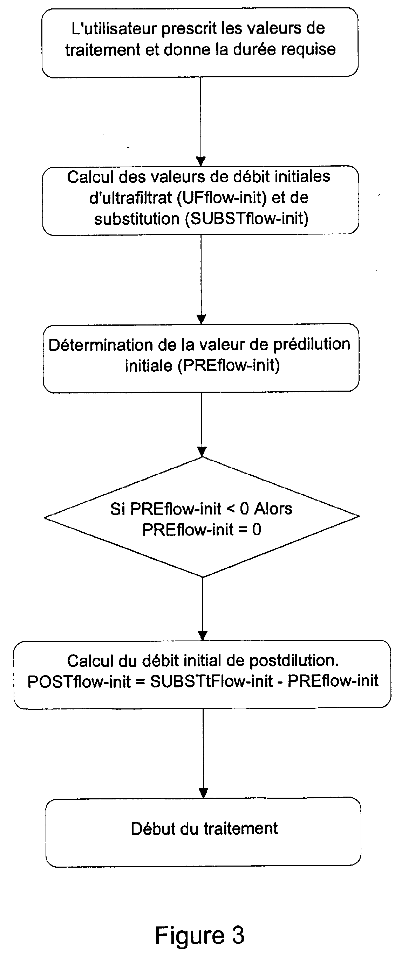

Comme décrit dans la suite d'opérations relatives à la mise en marche illustrée par la figure 3 du dispositif de circulation extracorporelle, l'opérateur adapte, préalablement au traitement proprement dit, le volume d'échange et la durée du traitement, le débit d'ultrafiltrat initial étant calculé en effectuant la division du volume par la durée prescrite. Le débit de liquide de substitution est adapté à celui d'ultrafiltrat corrigé des valeurs (pertes de poids souhaitées, apports et pertes externes) nécessaires pour maintenir ou adapter le poids du patient comme demandé par l'opérateur et scindé en deux selon un rapport initial entre la postdilution et la prédilution, déterminé sur des bases statistiques.

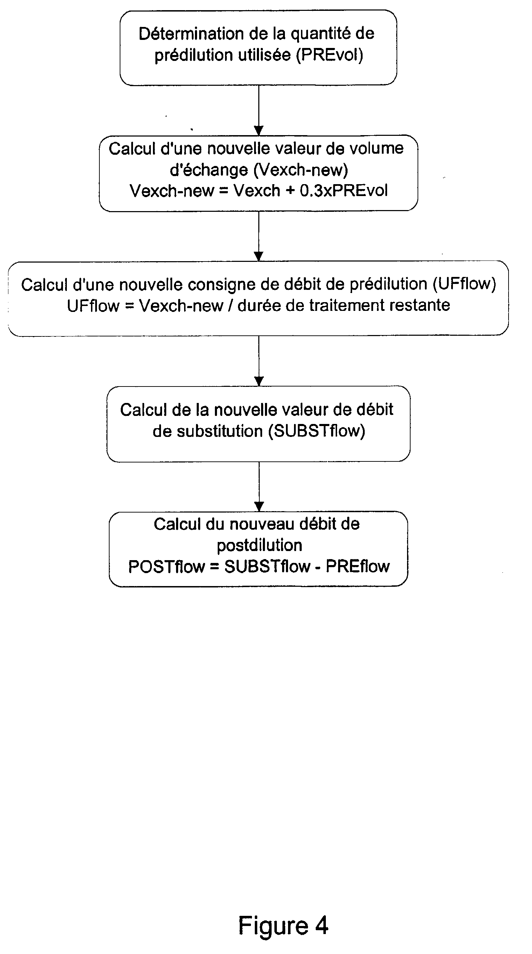

Comme illustré par la figure 4, durant le traitement, la quantité de liquide de substitution écoulée dans le conduit de prédilution 7' est mesurée ou calculée, sa valeur étant utilisée pour déterminer un nouveau volume d'échange correspondant à un degré d'épuration identique à celui déterminé par le volume initialement prescrit. Le débit d'ultrafiltrat 5 est ensuite adapté de façon à correspondre à ce nouveau volume d'échange, et si possible à atteindre les objectifs d'épuration et de variation de poids du patient dans la durée de traitement prescrite.

Les limites physiques du matériel sont enregistrées dans l'unité de calcul 9, comme par exemple les limites des moyens de commande des débits et la limite du rapport linéaire entre la baisse du degré d'épuration et la valeur de prédilution qui apparaît en augmentant le débit de liquide dans le conduit de prédilution 7'. Le point au-delà duquel le degré d'épuration est considéré comme insuffisant en regard du liquide de substitution consommé est déterminé par les variables de sang et de filtre intervenant dans la coagulation. Il peut être déterminé expérimentalement et enregistré dans l'unité de calcul 9 ou mesuré de manière continue, par exemple par dosage de l'urée et de sa variation dans l'ultrafiltrat 5. Pour déterminer si une limite physique est théoriquement dépassée, la valeur limite admissible du paramètre correspondant est mémorisée. Si une valeur limite admissible est dépassée, l'unité de calcul assigne la valeur du paramètre à sa valeur limite et prolonge la durée de traitement au-delà de la durée requise pour permettre d'atteindre les objectifs souhaités.

Etant donné que plus le débit sanguin extrait du patient est élevé, meilleures sont les conditions de filtration du dispositif, il peut être avantageux d'ajouter un capteur de débit 14 sur le conduit d'extraction 1 pour optimiser ce débit. A cet effet, l'unité de commande 19 de la pompe d'extraction du sang 2, augmente périodiquement la vitesse de cette pompe 2 jusqu'à ce que l'augmentation du débit de sang extrait n'augmente plus linéairement avec l'augmentation de la vitesse de la pompe 2, indiquant ainsi que l'on a dépassé le débit maximum admissible de l'accès vasculaire du patient. A ce moment, l'unité de commande 19 de la pompe à sang 2 diminue la vitesse de cette pompe 2 afin d'atteindre la dernière valeur connue pour être dans la plage linéaire.

Ce contrôle peut être réalisé de façon répétée durant le traitement de manière à maintenir le débit sanguin dans les conditions optimum de filtration. Le capteur de débit 14 sera avantageusement aussi utilisé pour déterminer de façon précise le débit sanguin.

Le débit d'anticoagulant peut être nul ou non, amené par un pousse-seringue auxiliaire ou par une pompe 18 commandée par l'unité de calcul 9.

Pour déterminer les débits d'une façon conforme à l'invention, l'unité de calcul 9 peut avoir l'architecture montrée sur la figure 2, l'invention ne se limitant pas à cet exemple.

Des données de configuration de l'appareil sont enregistrées initialement, généralement en dehors d'une séance thérapeutique, dans l'unité de calcul 9. Ces données peuvent comporter la définition des traitements standards, notamment le nom du traitement, le volume d'échange, la durée requise et les tubulures et filtres utilisés, et celles des apports et pertes de liquides externes à l'appareil notamment le type d'apport ou pertes, le débit par défaut et la masse à atteindre. Ces valeurs peuvent être modifiées durant le traitement, l'appareil prenant alors en compte les nouvelles valeurs.

En début de séance, l'opérateur indique les valeurs de traitement prescrites, par exemple le bilan hydrique du patient, la perte de poids désirée ou une perfusion. Si nécessaire, il modifie les valeurs définies initialement qui apparaissent par défaut. Cette mise en route du dispositif d'épuration est illustrée par le schéma de la figure 3. Le débit sanguin est défini selon l'une des méthodes décrite précédemment. L'unité de calcul 9 détermine alors le débit d'ultrafiltrat et de liquide de substitution initiaux, le taux de prédilution étant soit nul, soit calculé sur la base de règles préalablement définies et enregistrées dans l'appareil. Idéalement, le débit initial de prédilution sera proche de la valeur normale de fonctionnement à laquelle on s'attend. Le traitement commence et les pressions transfiltre et/ou transmembranaire sont mesurées de façon à déterminer en quelques secondes leurs valeurs normales de fonctionnement qui correspondent à un état stable du dispositif satisfaisant son fonctionnement selon les règles prédéterminées.

Durant le traitement, les débits des différentes pompes 2, 6, 8 et 8' sont ajustés automatiquement, soit à intervalles de temps réguliers, soit sur la base d'un événement déterminé comme par exemple une augmentation brutale de pression, en mesurant les valeurs de pression 10-13 et en comparant ces valeurs à celles considérées comme étant les valeurs normales de fonctionnement. On peut ainsi déterminer une variable à trois états qui définit notamment s'il faut réduire, augmenter ou maintenir le taux de prédilution.

Le taux de prédilution est ensuite ajusté en fonction de la valeur de la variable à trois états, puis le volume d'échange nécessaire est calculé, le débit d'ultrafiltrat étant adapté à cette nouvelle valeur.

Les règles permettant de déterminer ladite variable à trois états peuvent être définies de plusieurs façons, par exemple, on diminue le taux de prédilution à chaque cycle d'ajustement aussi longtemps que les pressions n'augmentent pas de plus de 10% par rapport à la valeur déterminée comme étant celle de fonctionnement, ou tant que les pressions ne présentent pas une augmentation de plus de 5% par 30 secondes. De la même façon on peut déterminer des critères de maintien et d'augmentation du taux de prédilution. Les valeurs de consigne des pompes 6 et 8' sont ensuite adaptés en tenant compte de la nouvelle valeur de consigne de prédilution.

Une variante consiste à fournir les mesures de pression 10-13 à l'unité 16 qui calcule alors les consignes des pompes 6, 8 et 8' en tenant compte des mesures et de règles de calcul préalablement enregistrées.

Il est également possible d'utiliser d'autres variables que les pressions. Les mesures de débit d'ultrafiltrat pour des vitesses de pompes ou des ouvertures de clamp données sont également des indicateurs du niveau d'encrassement du filtre.

Du fait que les paramètres qui influencent les niveaux normaux de pression évoluent au cours du traitement, il sera utile de les modifier au cours du traitement. Un moyen possible est de considérer tout niveau stable pendant au moins 5 minutes comme nouveau niveau normal de fonctionnement.

L'adaptation de la part de prédilution peut ne pas suffire à nettoyer suffisamment le filtre 4. Ainsi si ce dernier s'encrasse, ce qui est par exemple déterminé par une valeur de pression transmembranaire supérieure à une limite définie préalablement ou par une augmentation rapide de ladite pression, un nettoyage est réalisé en arrêtant la pompe d'extraction du sang 2, la pompe d'ultrafiltrat 6 et celle de postdilution 8' pendant une durée ou pour un volume ou une masse de liquide de restitution déterminée et en actionnant celle de prédilution 8 de façon à faire circuler de la solution physiologique à la place du sang et à rincer ainsi le filtre 4. La masse de solution injectée au patient durant le rinçage ainsi effectué est ensuite déduite lors de la poursuite du traitement.

Le bénéfice de l'invention est triple : Réduction de la quantité de solution médicale stérile de substitution coûteuse utilisée, suppression de l'encrassement de la circulation extracorporelle sans ajout obligatoire d'anticoagulants et ajustement automatique du volume d'échange pour obtenir le degré d'épuration souhaitée.

## Revendications

1. Dispositif d'épuration extracorporelle du sang comprenant un conduit d'extraction du sang (1), un conduit de retour du sang (3), des moyens de filtration du sang (4) situés entre le conduit d'extraction (1) et le conduit de retour (3), des moyens (2) pour faire circuler le sang, un conduit d'évacuation de l'ultrafiltrat (5) issu desdits moyens de filtration (4), des moyens (6) pour faire circuler l'ultrafiltrat dans ledit conduit d'évacuation (5), une source de solution de substitution (S), deux conduits de liaison (7', 7") entre cette source (S) et d'une part, ledit conduit d'extraction du sang (1), d'autre part, ledit conduit de retour du sang (3) et des moyens (8, 8') pour faire circuler la solution de substitution dans chacun desdits conduits de liaison (7', 7"), caractérisé en ce qu'il comporte en outre des moyens de mesure (10-13) d'au moins un des paramètres influencés par la résistance des moyens de filtration (4) à l'écoulement du liquide, des moyens (22) pour déterminer au moins une valeur de seuil dudit paramètre, des moyens (21) pour comparer ledit paramètre avec ladite valeur de seuil, des moyens de commande d'échange (16) pour calculer la répartition des débits respectifs à travers lesdits conduits de liaison (7', 7"), apte à réduire l'écart entre la valeur dudit paramètre mesuré et ladite valeur de seuil.

2. Dispositif selon la revendication 1, caractérisé en ce que lesdits moyens de mesure (10-13) d'au moins un des paramètres influencés par la résistance des moyens de filtration (4) à l'écoulement du liquide, mesurent au moins une valeur apte à déterminer la pression transmembranaire ou la pression transfiltre desdits moyens de filtration (4).

3. Dispositif selon la revendication 1, caractérisé en ce que lesdits moyens de mesure (10-13) d'au moins un des paramètres influencés par la résistance des moyens de filtration (4) à l'écoulement du liquide, mesurent le débit d'ultrafiltrat à travers le conduit d'évacuation (5).

4. Dispositif selon l'une des revendications précédentes, caractérisé en ce que lesdits moyens (22) pour déterminer au moins une valeur de seuil dudit paramètre, calculent cette valeur de seuil sur la base de plusieurs desdits paramètres mesurés ou de l'évolution dans le temps d'au moins un desdits paramètres.

5. Dispositif selon l'une des revendications précédentes, caractérisé en ce qu'il comporte des moyens (21) pour détecter le dépassement d'au moins une valeur de seuil, lesdits moyens de commande d'échange (16) étant programmés pour arrêter lesdits moyens (2, 6, 8') pour faire circuler le sang, l'ultrafiltrat et le liquide de substitution dans ledit conduit de liaison (7") reliant ladite source de liquide de substitution (S) audit conduit de retour (3), pendant que lesdits moyens de circulation (8) de liquide de substitution entre ladite source de liquide de substitution (S) et ledit conduit d'extraction (1) sont actionnés, jusqu'à ce qu'un volume, une masse de liquide de substitution ou une durée déterminée se soit écoulé.

6. Dispositif selon l'une des revendications 1-4, caractérisé en ce qu'il comporte des moyens (21) pour détecter le dépassement d'au moins une valeur de seuil, lesdits moyens de commande de prédilution (20) étant programmés pour modifier la valeur de consigne du débit des moyens de circulation du fluide (8) entre ladite source de liquide de substitution (S) et ledit conduit d'extraction du sang (1), consécutivement audit dépassement.

7. Dispositif selon l'une des revendications précédentes, caractérisé en ce que lesdits moyens de commande d'échange (16) sont programmés pour effectuer une compensation du volume d'échange, établie en fonction du volume ou de la masse de liquide de substitution injecté dans le conduit (7') reliant la source de liquide de substitution (S) au conduit d'extraction du sang (1).

8. Dispositif selon l'une des revendications précédentes, caractérisé en ce que les limites physiques de débits des moyens de circulation du liquide de substitution et de l'ultrafiltrat (6, 8, 8') sont enregistrées dans lesdits moyens de commande d'échange (16) et de prédilution (20) qui sont programmés pour prolonger la durée du traitement si les valeurs de traitement prescrites ne peuvent être atteintes sur la durée requise, compte tenu desdites limites physiques de débits enregistrées.

9. Dispositif selon l'une des revendications précédentes, caractérisé en ce que les moyens de commande d'échange (16) sont programmés pour calculer les consignes de débit pour les moyens de commande de prédilution (20), sur la base de règles enregistrées.

10. Dispositif selon l'une des revendications précédentes caractérisé en ce que ledit conduit d'extraction du sang (1) comporte un capteur de débit (14) connecté à l'unité de calcul (9) qui comporte des moyens (19) pour déterminer le débit maximum toléré par l'ensemble patient-accès vasculaire, en augmentant la vitesse de la pompe (2) d'extraction du sang et en comparant la linéarité de l'augmentation du débit avec l'augmentation de la vitesse de ladite pompe (2), et en diminuant la vitesse de ladite pompe (2) dès que le rapport débit/vitesse n'est plus linéaire, pour ramener la vitesse de la pompe (2) à la dernière valeur comprise dans la plage linéaire.
